(19) **Europäisches Patentamt / European Patent Office / Office européen des brevets**

(11) **EP 4 316 600 A1**

(12) **EUROPEAN PATENT APPLICATION**
published in accordance with Art. 153(4) EPC

(43) Date of publication:
**07.02.2024 Bulletin 2024/06**

(21) Application number: **22780534.8**

(22) Date of filing: **25.03.2022**

(51) International Patent Classification (IPC):
*A61Q 1/02* (2006.01)    *A61Q 1/06* (2006.01)
*A61Q 1/12* (2006.01)    *A61Q 17/04* (2006.01)
*A61K 8/19* (2006.01)    *A61K 8/25* (2006.01)
*A61K 8/29* (2006.01)    *A61K 8/37* (2006.01)
*A61K 8/63* (2006.01)

(52) Cooperative Patent Classification (CPC):
**A61K 8/19; A61K 8/25; A61K 8/29; A61K 8/37;
A61K 8/63; A61Q 1/02; A61Q 1/06; A61Q 1/12;
A61Q 17/04**

(86) International application number:
**PCT/JP2022/014232**

(87) International publication number:
**WO 2022/210320 (06.10.2022 Gazette 2022/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.03.2021 JP 2021060853**

(71) Applicant: **Matsumoto Trading CO., LTD.
Tokyo 103-0022 (JP)**

(72) Inventors:
• **NAKAGAWA Yuta**
  **Tokyo 103-0022 (JP)**
• **IGUCHI Risa**
  **Tokyo 103-0022 (JP)**

(74) Representative: **RCD-Patent
Giesen, Schmelcher & Griebel
Patentwanwälte PartG mbB
Postfach 3106
52118 Herzogenrath (DE)**

(54) **COMPOSITE POWDER AND COSMETIC CONTAINING SAME**

(57) Use of a composite powder characterized in that a plate-like powder is adhered to a spherical powder coated with an oil agent, and a cosmetic characterized by containing the composite powder provides a composite powder having texture, light diffusing properties, and an effect such as SPF enhancement similar to those of plastic microbeads, and a cosmetic blended with the composite powder.

FIG. 1

15kV 9.8mm x4.00k BSE L                    10.0μm

EP 4 316 600 A1

**Description**

Technical Field

The present invention relates to a composite powder to serve as an alternative for plastic microbeads and a cosmetic containing the same.

Background Art

Conventionally, plastic microbeads have been widely used in various formulations for the purpose of improving texture, imparting light diffusing properties, and imparting an SPF enhancing effect. Plastic microbeads are a powder, a film, or a fiber of a solid synthetic polymer (plastic) of 5 mm or less.

However, in recent years, the impact of plastic microbeads on the marine environment has been regarded as a problem, and a cosmetic in which the blending amount of plastic microbeads is reduced or plastic microbeads are not blended has been demanded.

As a cosmetic in which plastic microbeads are not blended, for example, a solid powder cosmetic characterized in that a cosmetic base material prepared by mixing a powder containing spherical nonporous silica with an oil agent is mixed with a solvent to form a slurry, and the slurry is filled in a container, and then, the solvent is removed is known (PTL 1).

However, this cosmetic has a problem that the texture is poor and the effect as a cosmetic is low as compared with cosmetics using plastic microbeads.

Citation List

Patent Literature

PTL 1: JP2020-93996A

Summary of Invention

Technical Problem

In view of the above circumstances, an object of the invention is to provide a composite powder having texture, light diffusing properties, and an effect such as SPF enhancement similar to those of plastic microbeads, and a cosmetic blended with the composite powder.

Solution to Problem

As a result of intensive studies to solve the above problem, the present inventors have found that a composite powder in which a plate-like powder is adhered to a spherical powder coated with an oil agent has texture, light diffusing properties, and an effect such as SPF enhancement similar to those of plastic microbeads, and thus completed the invention.

That is, the invention is directed to a composite powder characterized in that a plate-like powder is adhered to a spherical powder coated with an oil agent.

Further, the invention is directed to a cosmetic characterized by containing the composite powder.

Still further, the invention is directed to a method for producing a composite powder characterized by including the following steps (a) and (b):

(a) a step of mixing and heating an oil agent, a spherical powder, and a dispersion medium, thereby obtaining the spherical powder coated with the oil agent; and
(b) a step of stirring the spherical powder coated with the oil agent and a plate-like powder, thereby obtaining a composite powder in which the plate-like powder is adhered to the spherical powder coated with the oil agent.

Advantageous Effects of Invention

The composite powder of the invention has texture, light diffusing properties, and an effect such as SPF enhancement similar to those of plastic microbeads.

Therefore, a cosmetic containing the composite powder of the invention is equivalent to a conventional cosmetic blended with plastic microbeads, but has a low environmental impact because there is no need to incorporate plastic microbeads, and thus is superior to the conventional one.

Brief Description of Drawings

[FIG. 1] FIG. 1 is an electron micrograph of a composite powder obtained in Example 1 (4,000 times).
[FIG. 2] FIG. 2 is an electron micrograph of a composite powder obtained in Example 5 (3,000 times).
[FIG. 3] FIG. 3 is an evaluation sheet used for texture evaluation.

Description of Embodiments

The composite powder of the invention is a powder in which a plate-like powder is adhered to a spherical powder coated with an oil agent. Here, the phrase "is adhered" means that a state in which a plate-like powder is in contact with a surface of a spherical powder coated with an oil agent is observed with a scanning electron microscope. Further, the adhesion of a plate-like powder to a spherical powder coated with an oil agent may be partially or entirely.

The oil agent is not particularly limited as long as the spherical powder can be coated therewith, but for example, a semi-solid oil agent or a solid oil agent having no fluidity at ordinary temperature (15 to 25°C) is preferred. Examples of the semi-solid oil agent include hydrocarbon-based semi-solid oil agents, ester-based semi-solid oil agents, ether-based semi-solid oil agents, and silicone-based semi-solid oil agents, and for example, hydrocarbon-based semi-solid oil agents such as petrolatum, ester-based semi-solid oil agents such as dipentaerythrityl hexa(hydroxystearate/stearate/rosinate), dipentaerythrityl (hydroxystearate/stearate/rosinate), dipentaerythrityl hexahydroxystearate, dipentaerythrityl tetra(hydroxystearate/isostearate), dipentaerythrityl (hydroxystearate/isostearate), (caprylic/capric/myristic/stearic) triglyceride, bis-diglyceryl polyacyladipate-2, hydrogenated castor oil stearate, hydrogenated castor oil isostearate, hydrogenated castor oil hydroxystearate, hydrogenated palm oil, hydrogenated coconut oil, cholesteryl hydroxystearate, phytosteryl oleate, phytosteryl macadamia nut oil fatty acid, phytosteryl sunflower seed oil fatty acid, phytosteryl isostearate, (phytosteryl/isosteryl/cetyl/stearyl/behenyl) dimer dilinolate, dimer dilinoleyl bis(behenyl/isostearyl/phytosteryl) dimer dilinoleate, sucrose pentahydroxystearate, and di(octyldodecyl/phytosteryl/behenyl) lauroyl glutamate, ether-based semi-solid oil agents such as hydroxyalkyl dimer silinoleyl ether, and silicone-based semi-solid oil agents such as dimethicone crosspolymer and (dimethicone/vinyl dimethicone) crosspolymer.

In addition, as the solid oil agent, for example, a hydrocarbon-based solid oil agent, an ester-based solid oil agent, a higher alcohol-based solid oil agent, a silicone-based solid oil agent, or the like can be used. Examples thereof include hydrocarbon-based solid oil agents such as paraffin wax, ceresin wax, microcrystalline wax, polyethylene wax, ethylene/propylene copolymer, montan wax, and Fischer-Tropsch wax, ester-based solid oil agents such as hydrogenated jojoba oil, beeswax, carnauba wax, candelilla wax, rice wax, (eicosene/vinylpyrrolidone) copolymer, α-olefin/vinylpyrrolidone copolymer, and glyceryl tribehenate, higher alcohol-based solid oil agents such as stearyl alcohol, cetanol, lauryl alcohol, and behenyl alcohol, and silicone-based solid oil agents such as trimethylsiloxysilicate, polysilsesquioxane, (acrylates/dimethicone) copolymer, (acrylates/behenyl acrylate/dimethicone methacrylate) copolymer, (acrylates/stearyl acrylate/dimethicone methacrylate) copolymer, C30-45 alkyl methicone, stearyl dimethicone, stearoxytrimethylsilane, and C30-45 alkyldimethylsilyl polypropylsilsesquioxane.

Among these oil agents, ester-based semi-solid oil agents and ester-based solid oil agents are preferred, and ester-based semi-solid oil agents are more preferred. Further, these oil agents can be used alone or by combining two or more types thereof.

Since the composite powder of the invention is an alternative technique for plastic microbeads, the spherical powder is not particularly limited as long as it is not a plastic. Examples thereof include silica, titanium oxide, zinc oxide, aluminum oxide, calcium carbonate, magnesium carbonate, zirconium oxide, cerium oxide, calcium carbonate, barium sulfate, cellulose, and silk, and one in which the surface thereof is coated or encapsulated with titanium oxide, iron oxide, or the like may also be used, or it may be surface-treated with an oil agent, a silicone, an amino acid, or the like. Among these, silica, calcium carbonate, and barium sulfate are preferred, and silica is more preferred. These spherical powders can be used alone or by combining two or more types thereof. The size of the spherical powder is also not particularly limited, but the average particle diameter is 1 to 20 um, preferably 3 to 10 um. The average particle diameter is a value measured by electron microscopy or particle size distribution measurement with a laser diffraction/scattering method.

Since the composite powder of the invention is an alternative technique for plastic microbeads, the plate-like powder is not particularly limited as long as it is not a plastic. Examples thereof include titanium oxide, zirconium oxide, zinc oxide, cerium oxide, magnesium oxide, barium sulfate, calcium sulfate, magnesium sulfate, calcium carbonate, magnesium carbonate, iron oxide, carbon black, chromium oxide, chromium hydroxide, prussian blue, ultramarine, red iron oxide, talc, mica, kaolin, sericite, muscovite, synthetic mica , phlogopite, lepidolite, biotite, lithia mica, silica, aluminum silicate, magnesium silicate, magnesium aluminum silicate, calcium silicate, barium silicate, strontium silicate, a tungstate metal salt, hydroxyapatite, vermiculite, Higilite, bentonite, montmorillonite, hectorite, zeolite, a ceramic powder, dicalcium phosphate, alumina, aluminum hydroxide, boron nitride, bismuth oxychloride, titanium oxide-coated mica, titanium oxide-tin oxide-coated synthetic phlogopite, zinc oxide-coated mica, barium sulfate-coated mica, a titanium oxide-coated glass powder, a glass powder, an aluminum powder, silk, cellulose, crystalline cellulose, starch, and lauroyl lysine, and these may be surface-treated with an oil agent, a silicone, an amino acid, or the like. Among these, talc, mica, synthetic mica, and boron nitride are preferred, and synthetic mica and boron nitride are more preferred. These plate-like powders can be used alone or by combining two or more types thereof.

The size of the plate-like powder is also not particularly limited, but the average particle diameter is 50 um or less, preferably 1 to 20 um. The aspect ratio of the plate-like powder is also not particularly limited, but is 2 to 200, preferably 10 to 200. The average particle diameter is a value measured by electron microscopy or particle size distribution measurement with a laser diffraction/scattering method. Further, the aspect ratio is a value obtained by dividing the major axis of the plate-like powder by the thickness, and is generally obtained from electron microscopy of the powder. For example, it can be calculated using the average value of 10 to 20 samples of electron micrographs.

The mass ratio of the spherical powder, the plate-like powder, and the oil agent in the composite powder of the invention is not particularly limited, but is, for example, 99.8/0.1/0.1 to 1/1/1, preferably 98/1/1 to 75/20/5.

The composite powder of the invention has texture, light diffusing properties, and an effect such as SPF enhancement similar to those of plastic microbeads. Specifically, these are texture, light diffusing properties, and an effect such as SPF enhancement of a powder of nylon, polyacrylate, polyethylene, polystyrene, polyurethane, organopolysiloxane, or the like.

In addition, the composite powder of the invention has a first principal component score of -4.5 or less, preferably -5.5 or less and a second principal component score of 7.5 or less, preferably 6.0 or less, each of which is obtained by a principal component analysis of results of a sensory evaluation test of texture described in Examples. The principal component analysis refers to an analytical method for extracting two explanatory components with the highest correlation among factors for each texture evaluation item, and the first principal component score refers to a numerical value obtained by substituting an individual texture evaluation value for a first principal component obtained by the principal component analysis, and the second principal component score refers to a value obtained by substituting an individual texture evaluation value for a second principal component obtained by the principal component analysis.

The composite powder of the invention can be produced, for example, by including the following steps (a) and (b), preferably in the order of steps (a) and (b), but a production method other than the following steps may be used as long as the composite powder of the invention can be produced:

(a) a step of mixing and heating an oil agent, a spherical powder, and a dispersion medium, thereby obtaining the spherical powder coated with the oil agent; and

(b) a step of stirring the spherical powder coated with the oil agent and a plate-like powder, thereby obtaining a composite powder in which the plate-like powder is adhered to the spherical powder coated with the oil agent.

In the step (a), first, an oil agent, a spherical powder, and a dispersion medium are placed in a container and mixed with a disper mixer, a planetary mixer, a Henschel mixer, or the like. This produces a slurry.

The dispersion medium is not particularly limited, and examples thereof include alcohols such as isopropyl alcohol (IPA) and ethanol, water, n-hexane, and isoparaffin. Among these dispersion media, alcohols are preferred, and isopropyl alcohol is more preferred. The amount of the dispersion medium is not particularly limited, but may be, for example, the same amount as that of the spherical powder.

After mixing, the resultant is heated. When heating, it is preferred to perform mixing using a disper mixer, a planetary mixer, a Henschel mixer, or the like. Although the heating temperature and time are not particularly limited, a temperature and a time suitable for evaporating the dispersion medium from the slurry are preferred, and are for example, 80°C and 20 minutes or so when the dispersion medium is isopropyl alcohol. This coats the spherical powder with the oil agent.

In the step (b), the method of stirring the spherical powder coated with the oil agent and the plate-like powder is not particularly limited as long as the plate-like powder can be physically adhered to the spherical powder coated with the oil agent, and for example, a method capable of applying a physical force simultaneously with stirring such as a mortar, a jet mill, a ball mill, a bead mill, a pin mill, or a hammer mill is preferred. The stirring time is not particularly limited, but is 5 to 60 minutes, preferably 30 to 60 minutes.

After performing the steps (a) and (b), drying under reduced pressure, pulverization, classification, etc. may be further performed.

The composite powder of the invention can be used to replace plastic microbeads in a conventional cosmetic in which plastic microbeads are used. There is no need to incorporate plastic microbeads in the cosmetic containing the composite powder of the invention, and a cosmetic containing no plastic microbeads is formed, and therefore, the cosmetic has a low environmental impact. However, it goes without saying that the composite powder of the invention can be used in a cosmetic in combination with plastic microbeads.

Preferred examples of the cosmetic of the invention include makeup cosmetics such as a powder foundation, a liquid foundation, a blush, an eye shadow, and a lipstick that make use of the improvement of the texture, the light diffusing properties, and the like of the composite powder, and sunscreen cosmetics such as a sun care product and a base that make use of SPF enhancement.

Examples

Hereinafter, the invention will be described in detail with reference to Examples, but the invention is by no means limited to these Examples.

Examples 1 to 18 and Comparative Examples 1 to 15

Preparation of composite powder:

(1) Examples 1 to 18

In accordance with the formulations shown in Table 1, composite powders of Examples 1 to 18 were prepared by the following production method.

A) A spherical powder (component (A)) and isopropyl alcohol (IPA: dispersion medium) of the same mass as that of the spherical powder are uniformly mixed.
B) An oil agent (component (C)) is added to A and uniformly mixed.
C) B was heated and mixed at 80°C or higher, and IPA (dispersion medium) was volatilized until the slurry was converted into a powder form.
D) C was dried under reduced pressure, thereby obtaining the spherical powder coated with the oil agent.
E) The spherical powder coated with the oil agent and a plate-like powder (component (B)) are physically adhered to each other by grinding in a mortar for 30 minutes.

(2) Comparative Examples 1 to 3

In accordance with the formulations shown in Table 1, powders of Comparative Examples 1 to 4 were prepared by the following production method.

A) A spherical powder (component (A)) and isopropyl alcohol (IPA: dispersion medium) of the same mass as that of the spherical powder are uniformly mixed.
B) An oil agent (component (C)) is added to A and uniformly mixed.
C) B was heated and mixed at 80°C or higher, and IPA (dispersion medium) was volatilized until the slurry was converted into a powder form.
D) C was dried under reduced pressure, thereby obtaining the spherical powder coated with the oil agent.
E) The spherical powder coated with the oil agent and a plate-like powder (component (B)) are physically adhered to each other by grinding in a mortar for 30 minutes.

(3) Comparative Examples 4 to 6

In accordance with the formulations shown in Table 1, powders of Comparative Examples 4 to 6 were prepared by the following production method.

A) A spherical powder (component (A)) and isopropyl alcohol (IPA: dispersion medium) of the same mass as that of the spherical powder are uniformly mixed.
B) An oil agent (component (C)) is added to A and uniformly mixed.
C) B was heated and mixed at 80°C or higher, and IPA (dispersion medium) was volatilized until the slurry was converted into a powder form.
D) C was dried under reduced pressure, thereby obtaining the spherical powder coated with the oil agent.

(4) Comparative Example 14

In accordance with the formulation shown in Table 1, a powder of Comparative Example 14 was prepared by the following production method.

A) A spherical powder and a plate-like powder are uniformly mixed.
B) An oil agent is heated to 70°C or higher and added to A.
C) B is mixed for 30 seconds with a mixer.

(5) Comparative Example 15

In accordance with the formulation shown in Table 1, a powder of Comparative Example 15 was prepared by the following production method.

A) A spherical powder and a plate-like powder are uniformly mixed.
B) B is mixed for 30 seconds with a mixer.

In Comparative Examples 7 to 13, a spherical powder (corresponding to plastic microbeads in Comparative Examples 7 to 10) was used as it was.

[Table 1-1]

| Compo-nent | | Exam-ple 1 | Exam-ple 2 | Exam-ple 3 | Exam-ple 4 | Compara-tive Ex-ample 1 | Compara-tive Ex-ample 2 | Compara-tive Ex-ample 3 | Exam-ple 5 | Exam-ple 6 | Exam-ple 7 | Exam-ple 8 | Exam-ple 9 | Exam-ple 10 | Exam-ple 11 | Exam-ple 12 | Exam-ple 13 | Exam-ple 14 |
|---|---|---|---|---|---|---|---|---|---|---|---|---|---|---|---|---|---|---|
| (A) | Iron oxide/titanium oxide-coated silica*1 (average particle diameter: 6 μm) | 88.2 | 88.2 | 88.2 | 88.2 | 88.2 | 88.2 | 88.2 | 88.2 | 88.2 | 88.2 | 88.2 | 88.2 | 88.2 | 88.2 | 88.2 | | 88.2 |
| | Silica (average particle diameter: 5 μm) | | | | | | | | | | | | | | | | | |
| | Silica (average particle diameter: 4 μm) | | | | | | | | | | | | | | | | 86.4 | |
| | Silicone-treated silica*2 (average particle diameter: 5 μm) | | | | | | | | | | | | | | | | | |
| A1 | (HDI/trimethylol-hexyllactone) crosspolymer (average particle diameter: 15 μm) | | | | | | | | | | | | | | | | | |
| | Nylon-12 (aver-age particle di-ameter: 12 μm) | | | | | | | | | | | | | | | | | |
| | Polymethyl meth-acrylate (average particle diameter: 8 μm) | | | | | | | | | | | | | | | | | |

EP 4 316 600 A1

7

| Component | | Example 1 | Example 2 | Example 3 | Example 4 | Comparative Example 1 | Comparative Example 2 | Comparative Example 3 | Example 5 | Example 6 | Example 7 | Example 8 | Example 9 | Example 10 | Example 11 | Example 12 | Example 13 | Example 14 |
|---|---|---|---|---|---|---|---|---|---|---|---|---|---|---|---|---|---|---|
| | (Vinyl dimethicone/methicone silsesquioxane) crosspolymer (average particle diameter: 5 μm) | | | | | | | | | | | | | | | | | |
| (B) | Synthetic fluorophlogopite (average particle diameter: 12 μm, aspect ratio: 20) | 10.0 | 100 | 100 | 100 | 100 | 100 | 10.0 | | | | | | | | | | |
| | Synthetic fluorophlogopite (average particle diameter: 40 μm, aspect ratio: 90) | | | | | | | | 100 | | | | | | | | | |
| | Boron nitride (average particle diameter: 6 μm, aspect ratio: 10) | | | | | | | | | 100 | | | | | | | | |
| | Talc (average particle diameter: 2 μm, aspect ratio: 4) | | | | | | | | | | 100 | | | | | | | |
| | Mica (average particle diameter: 10 μm, aspect ratio: 70) | | | | | | | | | | | 100 | | | | | | |

EP 4 316 600 A1

8

(continued)

| Compo-nent | | Exam-ple 1 | Exam-ple 2 | Exam-ple 3 | Exam-ple 4 | Compara-tive Ex-ample 1 | Compara-tive Ex-ample 2 | Compara-tive Ex-ample 3 | Exam-ple 5 | Exam-ple 6 | Exam-ple 7 | Exam-ple 8 | Exam-ple 9 | Exam-ple 10 | Exam-ple 11 | Exam-ple 12 | Exam-ple 13 | Exam-ple 14 |
|---|---|---|---|---|---|---|---|---|---|---|---|---|---|---|---|---|---|---|
| | Sericite (average particle diameter: 12 $\mu$m, aspect ratio: 15) | | | | | | | | | | | | 100 | | | | | |
| | Lauroyl lysine (average particle diameter: 12 $\mu$m, aspect ratio: 38) | | | | | | | | | | | | | 100 | | | | |
| | Mica (average particle diameter: 42 $\mu$m, aspect ratio: 80) | | | | | | | | | | | | | | 100 | | | |
| | Mica (average particle diameter: 18 $\mu$m, aspect ratio: 150) | | | | | | | | | | | | | | | 100 | | |
| | Amino acid-treated talc*3 (average particle diameter: 6 $\mu$m, aspect ratio: 18) | | | | | | | | | | | | | | | | 100 | |
| | Silicone-treated talc*4 (average particle diameter: 9 $\mu$m, aspect ratio: 50) | | | | | | | | | | | | | | | | | 10.0 |

9

EP 4 316 600 A1

| Compo-nent | | Exam-ple 1 | Exam-ple 2 | Exam-ple 3 | Exam-ple 4 | Compara-tive Ex-ample 1 | Compara-tive Ex-ample 2 | Compara-tive Ex-ample 3 | Exam-ple 5 | Exam-ple 6 | Exam-ple 7 | Exam-ple 8 | Exam-ple 9 | Exam-ple 10 | Exam-ple 11 | Exam-ple 12 | Exam-ple 13 | Exam-ple 14 |
|---|---|---|---|---|---|---|---|---|---|---|---|---|---|---|---|---|---|---|
| (C) | Dimer dilinoleyl bis(behenyl/iso-stearyl/phytos-teryl) dimer dili-noleate*5 (melting point: 40°C) | 1.8 | | | | | | | 1.8 | 1.8 | 1.8 | 1.8 | 1.8 | 1.8 | 1.8 | 1.8 | 3.6 | 1.8 |
| | Candelilla wax (melting point: 71°C) | | 1.8 | | | | | | | | | | | | | | | |
| | Dipentaerythrityl hexa(hydroxystearate/stearate/rosinate)(melting point: 37°C) | | | 1.8 | | | | | | | | | | | | | | |
| | Petrolatum (melting point: 50 to 60°C) | | | | 1.8 | | | | | | | | | | | | | |
| | Diisostearyl malate | | | | | 1.8 | | | | | | | | | | | | |
| | Squalane | | | | | | 1.8 | | | | | | | | | | | |
| | Cetyl ethylhexanoate | | | | | | | 1.8 | | | | | | | | | | |
| Total | | 100.0 | 100.0 | 100.0 | 100.0 | 100.0 | 100.0 | 1000 | 100.0 | 100.0 | 100.0 | 100.0 | 100.0 | 100.0 | 100.0 | 100.0 | 100.0 | 1000 |
| Total (A) | | 88.2 | 88.2 | 88.2 | 88.2 | 88.2 | 88.2 | 88.2 | 88.2 | 88.2 | 88.2 | 88.2 | 88.2 | 88.2 | 88.2 | 88.2 | 86.4 | 88.2 |
| Total (B) | | 100 | 100 | 100 | 100 | 100 | 100 | 10.0 | 100 | 100 | 100 | 100 | 100 | 100 | 100 | 100 | 100 | 10.0 |
| Total (C) | | 1.8 | 1.8 | 1.8 | 1.8 | 1.8 | 1.8 | 1.8 | 1.8 | 1.8 | 1.8 | 1.8 | 1.8 | 1.8 | 1.8 | 1.8 | 3.6 | 1.8 |

EP 4 316 600 A1

(continued)

| Component | | Example 1 | Example 2 | Example 3 | Example 4 | Comparative Example 1 | Comparative Example 2 | Comparative Example 3 | Example 5 | Example 6 | Example 7 | Example 8 | Example 9 | Example 10 | Example 11 | Example 12 | Example 13 | Example 14 |
|---|---|---|---|---|---|---|---|---|---|---|---|---|---|---|---|---|---|---|
| Evaluation | First principal component score (smooth-rough) | -7.29 | -5.32 | -5.56 | -5.65 | -4.14 | -4.11 | -4.20 | -5.03 | -5.77 | -5.61 | -6.61 | -5.55 | -4.84 | -4.91 | -5.87 | -9.09 | -9.42 |
| | Second principal component score (wet-dry) | 6.00 | 6.31 | 5.82 | 5.32 | 5.53 | 5.93 | 6.45 | 5.95 | 5.69 | 5.48 | 6.48 | 6.05 | 4.73 | 6.39 | 5.99 | 5.16 | 5.40 |

*1 RonaFlair Flawless (Merck Performance Materials)
*2 SA-SB-300 (Miyoshi Kasei)
*3 NAI-talc JA-13R (Miyoshi Kasei)
*4 SA-talc JA-46R (Miyoshi Kasei)
*5 Plandool-G (Nippon Fine Chemical)

[Table 1-2]

| Component | | Comparative Example 4 | Example 15 | Example 16 | Example 17 | Example 18 | Comparative Example 5 | Comparative Example 6 | Comparative Example 7 | Comparative Example 8 | Comparative Example 9 | Comparative Example 10 | Comparative Example 11 | Comparative Example 12 | Comparative Example 13 | Comparative Example 14 | Comparative Example 15 |
|---|---|---|---|---|---|---|---|---|---|---|---|---|---|---|---|---|---|
| (A) | Iron oxide/titanium oxide-coated silica*1 (average particle diameter: 6 μm) | 98.0 | | | | | | | | | | | 100.0 | | | 88.2 | 900 |
| | Silica (average particle diameter: 5 μm) | | | 88.2 | 73.4 | | | 98.0 | | | | | | 100.0 | | | |
| | Silica (average particle diameter: 4 μm) | | 86.4 | | | | 96.0 | | | | | | | | 1000 | | |
| | Silicone-treated silica*2 (average particle diameter: 5 μm) | | | | | 87.3 | | | | | | | | | | | |
| | (HDI/trimethylolhexyllactone) crosspolymer (average particle diameter: 15 μm) | | | | | | | | 100.0 | | | | | | | | |
| | Nylon-12 (average particle diameter: 12 μm) | | | | | | | | | 100.0 | | | | | | | |
| | Polymethyl methacrylate (average particle diameter: 8 μm) | | | | | | | | | | 1000 | | | | | | |

(continued)

| Component | | Comparative Example 4 | Example 15 | Example 16 | Example 17 | Example 18 | Comparative Example 5 | Comparative Example 6 | Comparative Example 7 | Comparative Example 8 | Comparative Example 9 | Comparative Example 10 | Comparative Example 11 | Comparative Example 12 | Comparative Example 13 | Comparative Example 14 | Comparative Example 15 |
|---|---|---|---|---|---|---|---|---|---|---|---|---|---|---|---|---|---|
| | (Vinyl dimethicone/methicone silsesquioxane) crosspolymer (average particle diameter: 5 μm) | | | | | | | | | | | | | | | | |
| (B) | Synthetic fluorophlogopite (average particle diameter: 12 μm, aspect ratio: 20) | | 10.0 | 100 | 8.3 | | | | | | | 1000 | | | | 10.0 | 100 |
| | Synthetic fluorophlogopite (average particle diameter: 40 μm, aspect ratio: 90) | | | | | | | | | | | | | | | | |
| | Boron nitride (average particle diameter: 6 μm, aspect ratio: 10) | | | | | 100 | | | | | | | | | | | |
| | Talc (average particle diameter: 2 μm, aspect ratio: 4) | | | | | | | | | | | | | | | | |

(continued)

| Compo- nent | Compar- ative Ex- ample 4 | Exam- ple 15 | Exam- ple 16 | Exam- ple 17 | Exam- ple 18 | Compar- ative Ex- ample 5 | Compar- ative Ex- ample 6 | Compar- ative Ex- ample 7 | Compar- ative Ex- ample 8 | Compar- ative Ex- ample 9 | Compar- ative Ex- ample 10 | Compar- ative Ex- ample 11 | Compar- ative Ex- ample 12 | Compar- ative Ex- ample 13 | Compar- ative Ex- ample 14 | Compar- ative Ex- ample 15 |
|---|---|---|---|---|---|---|---|---|---|---|---|---|---|---|---|---|
| Mica (average particle diame- ter: 10 μm, as- pect ratio: 70) | | | | | | | | | | | | | | | | |
| Sericite (aver- age particle di- ameter: 12 μm, aspect ratio: 15) | | | | | | | | | | | | | | | | |
| Lauroyl lysine (average parti- cle diameter: 12 μm, aspect ra- tio: 38) | | | | | | | | | | | | | | | | |
| Mica (average particle diame- ter: 42 μm, as- pect ratio: 80) | | | | | | | | | | | | | | | | |
| Mica (average particle diame- ter: 18 μm, as- pect ratio: 150) | | | | | | | | | | | | | | | | |
| Amino acid- treated talc*3 (average parti- cle diameter: 6 μm, aspect ra- tio: 18) | | | | | | | | | | | | | | | | |

| Compo-nent | | Comparative Example 4 | Example 15 | Example 16 | Example 17 | Example 18 | Comparative Example 5 | Comparative Example 6 | Comparative Example 7 | Comparative Example 8 | Comparative Example 9 | Comparative Example 10 | Comparative Example 11 | Comparative Example 12 | Comparative Example 13 | Comparative Example 14 | Comparative Example 15 |
|---|---|---|---|---|---|---|---|---|---|---|---|---|---|---|---|---|---|
| | Silicone-treated talc*4 (average particle diameter: 9 μm, aspect ratio: 50) | | | | | | | | | | | | | | | | |
| (C) | Dimer dilinoleyl bis(behenyl/isostearyl/phytosteryl) dimer dilinoleate*5 (melting point: 40°C) | 2.0 | 3.6 | 1.8 | 18.3 | 2.7 | 4.0 | 20 | | | | | | | | 1.8 | |
| | Candelilla wax (melting point: 71°C) | | | | | | | | | | | | | | | | |
| | Dipentaerythrityl hexa(hydroxystearate/stearate/rosinate) (melting point: 37°C) | | | | | | | | | | | | | | | | |
| | Petrolatum (melting point: 50 to 60°C) | | | | | | | | | | | | | | | | |
| | Diisostearyl malate | | | | | | | | | | | | | | | | |
| | Squalane | | | | | | | | | | | | | | | | |
| | Cetyl ethylhexanoate | | | | | | | | | | | | | | | | |
| Total | | 100.0 | 1000 | 100.0 | 1000 | 100.0 | 1000 | 1000 | 100.0 | 100.0 | 1000 | 1000 | 100.0 | 100.0 | 1000 | 1000 | 100.0 |

EP 4 316 600 A1

| Component | | Comparative Example 4 | Example 15 | Example 16 | Example 17 | Example 18 | Comparative Example 5 | Comparative Example 6 | Comparative Example 7 | Comparative Example 8 | Comparative Example 9 | Comparative Example 10 | Comparative Example 11 | Comparative Example 12 | Comparative Example 13 | Comparative Example 14 | Comparative Example 15 |
|---|---|---|---|---|---|---|---|---|---|---|---|---|---|---|---|---|---|
| Total(A) | | 98.0 | 86.4 | 88.2 | 73.4 | 87.3 | 96.0 | 98.0 | 100.0 | 100.0 | 1000 | 1000 | 100.0 | 100.0 | 1000 | 88.2 | 900 |
| Total(B) | | 0.0 | 10.0 | 100 | 8.3 | 100 | 00 | 00 | 00 | 00 | 00 | 00 | 00 | 00 | 00 | 10.0 | 100 |
| Total(C) | | 2.0 | 3.6 | 1.8 | 18.3 | 2.7 | 4.0 | 2.0 | 00 | 00 | 00 | 00 | 00 | 00 | 0.0 | 1.8 | 00 |
| Evaluation | First principal component score (smooth-rough) | -1.68 | -8.79 | -5.56 | -6.10 | -8.99 | -4.39 | -4.86 | -6.59 | -7.41 | -7.86 | -10.35 | -2.40 | -5.22 | -7.63 | -4.26 | -0.38 |
| | Second principal component score (wet-dry) | 6.70 | 5.40 | 7.32 | 5.93 | 5.89 | 6.93 | 8.50 | 6.07 | 5.97 | 6.33 | 5.38 | 6.95 | 7.58 | 7.79 | 6.56 | 7.11 |

*1 RonaFlair Flawless (Merck Performance Materials)
*2 SA-SB-300 (Miyoshi Kasei)
*3 NAI-talc JA-13R (Miyoshi Kasei)
*4 SA-talc JA-46R (Miyoshi Kasei)
*5 Plandool-G (Nippon Fine Chemical)

<Texture Evaluation>

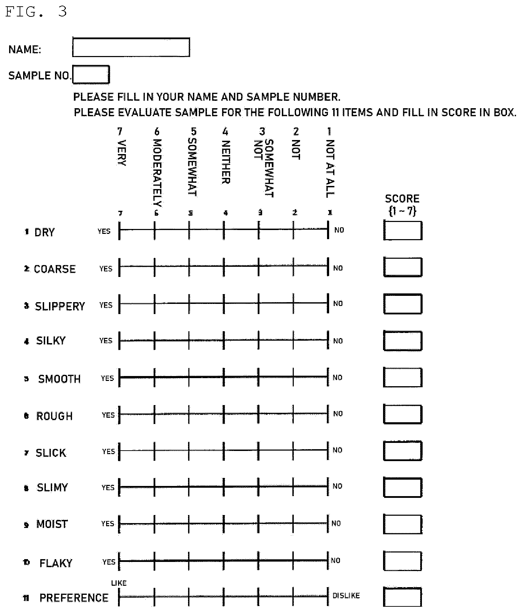

The texture evaluation of the composite powders of Examples 1 to 18 and the powders of Comparative Examples 1 to 15 obtained above was performed according to the following method using the evaluation sheet shown in FIG. 3 by a panel of 7 cosmetic specialists. This result is also shown in Table 1.

(Evaluation Method)

The powder prepared above was randomly touched in a blind manner and evaluated on a 7-point scale from 1 to 7 using the SD method with respect to the following 10 items: "dry", "coarse", "slippery", "silky", "smooth", "rough", "slick", "slimy", "flaky", and "moist", and a principal component analysis was performed based on the average value of the evaluation scores given by 7 specialists.

The principal component analysis was performed using the average score of the evaluation results of each sample. The cumulative contribution ratios of the first principal component and the second principal component obtained were 86%, which indicates that the two components have a sufficient explanatory power. The powders corresponding to plastic microbeads (Comparative Examples 7 to 10) had a first principal component score of -4.5 or less and a second principal component score of 7.5 or less, and all the Examples of the invention fell within the ranges (that is, the texture was equivalent to that of plastic microbeads). On the other hand, in the case of the spherical powder only coated with the oil agent, the non-composite powder, or the simply mixed powder, the scores did not fall within the ranges.

The principal component analysis is a known method that is also used in JP6868468B, JP6667607B, JP6879276B, JP6617767B, and the like. Specifically, when the principal component analysis is performed according to the description in [0036] to [0048] in JP6868468B, the method is as follows.

First, a principal component u is represented by the following formula 1. Here, the principal component u becomes a small number of characteristic variables representing the characteristics of a data group from multivariate data (also referred to as synthetic variables).

[Math. 1]

$$u_i = a_1 x_{1,i} + a_2 x_{2,i} + \ldots + a_{n-1} x_{n-1,i} + a_n x_{n,i} \ldots \text{formula 1}$$

Provided that n is the number of variables (the number of evaluation items in the present application), i is a natural number, x is the data of each variable (score of each evaluation item), $a_2$, $a_2$, ... $a_{n-1}$, and $a_n$ are the coefficients of the principal component.

The coefficients of the principal component $a_2$, $a_2$, ... $a_{n-1}$, and $a_n$ are obtained so that the variance of the principal component u is maximized. However, the coefficients of the principal component satisfy the relationship of the following formula 2.

[Math. 2]

$$a_1^2 + a_2^2 + \ldots + a_{n-1}^2 + a_n^2 = 1 \ldots \text{formula 2}$$

In order to obtain the coefficients of the principal component $a_1$, $a_2$, ... $a_{n-1}$, and $a_n$, first, the variance/covariance matrix of the original data group is calculated, and the eigenvalue problem of the variance/covariance matrix is solved. Eigenvectors that are solutions of the eigenvalue problem correspond to the coefficients $a_1$, $a_2$, ... $a_{n-1}$, and $a_n$. In addition, the obtained principal components are n sets (10 sets of the number of evaluation items in the present application), and are called a first principal component, a second principal component, and an $n_{th}$ principal component in descending order of the eigenvalue, and in the present application, the first principal component and the second principal component are used.

It is possible to ascertain the texture characteristics of the powder raw material by obtaining the principal component score using the eigenvector obtained from the principal component analysis by a known method as described above.

The first principal component score was calculated according to such a known method as follows: "first principal component score = "dry" score × "dry" first principal component eigenvector + "flaky" score × "flaky" first principal component eigenvector + "rough" score × "rough" first principal component eigenvector + "coarse" score × "coarse" first principal component eigenvector + "slippery" score × "slippery" first principal component eigenvector + "silky" score × "silky" first principal component eigenvector + "slick" score × "slick" first principal component eigenvector + "smooth" score × "smooth" first principal component eigenvector + "slimy" score × "slimy" first principal component eigenvector + "moist" score × "moist" first principal component eigenvector".

Here, the first principal component score is the sum of the product of the eigenvector of each evaluation item obtained by the principal component analysis and the score of each evaluation item. Further, the second principal component score was calculated in the same manner as the first principal component score.

In the present application, the powder is evaluated on a 7-point scale from 1 to 7 using the SD method with respect to the following 10 items: "dry", "coarse", "slippery", "silky", "smooth", "rough", "slick", "slimy", "flaky", and "moist", and the principal component analysis is performed based on the average value of the evaluation scores given by 7 specialists. In this principal component analysis, "First principal component score (smooth-rough)" and "Second principal component score (wet-dry)" are described in the evaluation columns in Tables 1-1 and 1-2, and this indicates that the first principal component is highly correlated with "smooth", "slick", "coarse", and "rough" associated with the evaluation of smooth-rough of the powder, and the second principal component is highly correlated with "dry" and "moist" associated with the evaluation of wet-dry of the powder.

<Exterior Photograph>

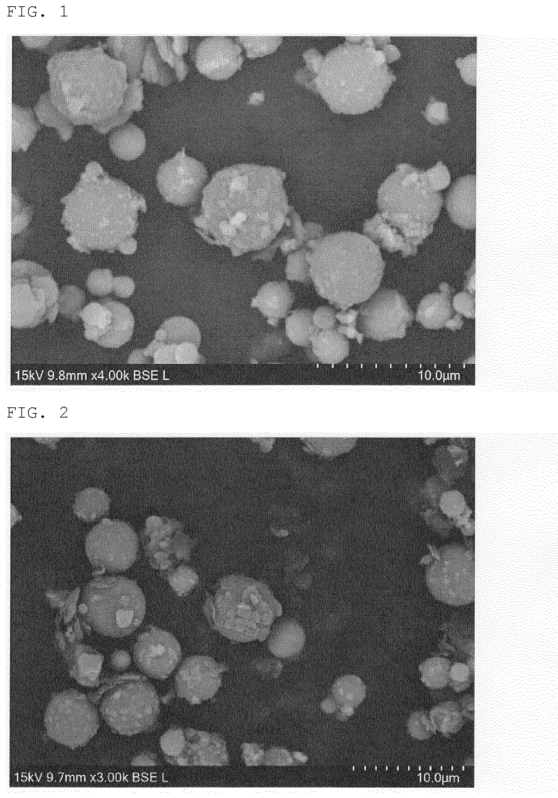

An electron micrograph of the composite powder of Example 1 obtained above is shown in FIG. 1, and an electron micrograph of the composite powder of Example 6 is shown in FIG. 2. The composite powders were powders in which a plate-like powder is partially adhered to a spherical powder coated with an oil agent.

The composite powders of the invention were all powders in which a plate-like powder is adhered to a spherical powder coated with an oil agent.

Example 19 and Comparative Examples 16 to 18

Powder foundation:

Powder foundations were prepared according to the following production method using the formulation shown in the following Table 2.

A) 1 to 14 are mixed with a mixer.
B) 15 to 18 are melted by heating and uniformly mixed.
C) B is added to A and mixed with a mixer.
D) C is pulverized, filled into a container, and then molded.

[Table 2]

| | | Raw material name | | Example 19 | Comparative Example 16 | Comparative Example 17 | Comparative Example 18 |
|---|---|---|---|---|---|---|---|
| | 1 | NAI-talc JA-46R (Miyoshi Kasei) | Amino acid-treated talc | 42.5 | 42.5 | 42.5 | 42.5 |
| | 2 | Powder base L (NOF) | Zinc laurate | 5 | 5 | 5 | 5 |
| | 3 | RonaFlair Boroneige SF-6 (Merck Performance Materials) | Boron nitride | 15 | 15 | 15 | 15 |
| | 4 | RonaFlair SynMica M (Merck Performance Materials) | Synthetic fluorophlogopite | 5.5 | 5.5 | 5.5 | 5.5 |
| | 5 | plate barium sulfate HM (Sakai Chemical Industry) | Barium sulfate | 10 | 10 | 10 | 10 |
| | 6 | SA-titanium CR-50 (Miyoshi Kasei) | Silicone-treated titanium oxide | 7.2 | 7.2 | 7.2 | 7.2 |

(continued)

| | Raw material name | | Example 19 | Comparative Example 16 | Comparative Example 17 | Comparative Example 18 |
|---|---|---|---|---|---|---|
| 7 | SA-yellow LL-100P (Miyoshi Kasei) | Silicone-treated yellow iron oxide | 1.6 | 1.6 | 1.6 | 1.6 |
| 8 | SA-red R-516PS (Miyoshi Kasei) | Silicone-treated red iron oxide | 0.8 | 0.8 | 0.8 | 0.8 |
| 9 | SA-black BL-100P (Miyoshi Kasei) | Silicone-treated black iron oxide | 0.4 | 0.4 | 0.4 | 0.4 |
| 10 | | Composite powder of Example 1 | 6 | | | |
| 11 | | Composite powder of Comparative Example 4 | | 6 | | |
| 12 | RonaFlair Flawless (Merck Performance Materials) | Iron oxide/titanium oxide-coated silica (average particle diameter: 6 $\mu$m) | | | 6 | 5.29 |
| 13 | | Synthetic fluorophlogopite (average particle diameter: 12 $\mu$m) | | | | 0.6 |
| 14 | Plandool-G (Nippon Fine Chemical) | Dimer dilinoleyl bis (behenyl/isostearyl/ phytosteryl) dimer dilinoleate | | | | 0.11 |
| 15 | DOWSIL SH 200 C Fluid 100cSt (Dow Toray) | Dimethicone | 2.8 | 2.8 | 2.8 | 2.8 |
| 16 | FineNeo-IOTG (Nippon Fine Chemical) | Triethylhexanoin | 1 | 1 | 1 | 1 |
| 17 | squalane | Squalane | 2 | 2 | 2 | 2 |
| 18 | Plandool-H (Nippon Fine Chemical) | (Phytosteryl/isostearyl/ cetyl/stearyl/behenyl) dimer dilinolate | 0.2 | 0.2 | 0.2 | 0.2 |
| | Total | | 100 | 100 | 100 | 100 |
| | Spreadability upon application | | A | D | C | C |
| | Smoothness upon application | | A | D | D | D |
| | Moist texture upon application | | A | C | D | D |
| | Wrinkle blurring effect | | A | C | C | C |

<Usability Evaluation>

A panel of 5 cosmetic specialists was asked to use these powder foundations and evaluate spreadability upon application, smoothness upon application, a moist texture after application, and a wrinkle blurring effect to score 1 point in the case of being evaluated as poor and to score 5 points in the case of being evaluated as good. The average score of the panel was calculated and determined according to the determination criteria shown below. This result is also shown in Table 2.

(Determination Criteria)

| [Average Score] | [Determination] |
|---|---|
| 4.5 or more: | A |
| 3.5 or more and less than 4.5: | B |
| 2 or more and less than 3.5: | C |
| Less than 2: | D |

The obtained powder foundation had excellent spreadability, smoothness, and moist texture upon application, and had a good wrinkle blurring effect.

Example 20 and Comparative Example 19

Sun care product:

Sun care products were prepared according to the following production method using the formulation shown in the following Table 3.

A) 1 to 4 are heated and uniformly mixed.
B) 5 to 8 are uniformly mixed with a homomixer (5000 rpm, 3 minutes).
C) 9 to 12 are uniformly mixed.
D) B is added to A and uniformly mixed.
E) C is gradually added to D and emulsified with a homomixer (5000 rpm, 3 minutes)
F) 13 to 14 are added to E and uniformly mixed.

[Table 3]

| | | | Example 20 | Comparative Example 19 |
|---|---|---|---|---|
| 1 | Uvinul A Plus B (BASF) | Diethylamino hydroxybenzoyl hexyl benzoate, ethylhexyl methoxycinnamate | 3.00 | 3.00 |
| 2 | Tinosorb S (BASF) | Bis-ethylhexyloxyphenol methoxyphenyl triazine | 0.50 | 0.50 |
| 3 | Uvinul MC 80 N (BASF) | Ethylhexyl methoxycinnamate | 1.50 | 1.50 |
| 4 | FineNee-iPSE (Nippon Fine Chemical) | Diisopropyl sebacate | 20.00 | 20.00 |
| 5 | PolyAquol OS2 (Innovacos) | Polyglyceryl-2 oleate, polyhydroxystearic acid, polyglyceryl-2 stearate | 3.00 | 3.00 |
| 6 | BENTONE GEL ISD V (Elementis) | Disteardimonium hectorite, isododecane, propylene carbonate | 6.00 | 6.00 |
| 7 | | Isododecane | 22.20 | 22.20 |
| 8 | DOWSIL VM-2270 Aerogel Fine Particle (Dow Toray) | Silica silylate | 1.00 | 1.00 |
| 9 | | Water | 19.00 | 22.00 |
| 10 | | BG | 5.00 | 5.00 |

(continued)

|  | | | Example 20 | Comparative Example 19 |
|---|---|---|---|---|
| 11 | | Sodium chloride | 0.30 | 0.30 |
| 12 | PHENOXETOL (Clariant Japan) | Phenoxyethanol | 0.50 | 0.50 |
| 13 | | Composite powder of Example 1 | 3.00 | |
| 14 | DIF-OP-3W (Sakai Chemical Industry) | Zinc oxide, hydrated silica, hydrogen dimethicone, ethylhexyl palmitate, polyhydroxystearic acid | 10.00 | 10.00 |
| 15 | DIS-OP-10A (Sakai Chemical Industry) | Titanium oxide, hydrated silica, aluminum hydroxide, hydrogen dimethicone, ethylhexyl palmitate, polyhydroxystearic acid | 5.00 | 5.00 |
| | Total | | 100.00 | 100.00 |
| | Spreadability upon application | | A | C |
| | Smoothness upon application | | A | D |
| | SPF(In vitro) | | 95.3 | 81.8 |

<Usability Evaluation>

These sun care products were evaluated for spreadability upon application and smoothness upon application in the same manner as in the above Examples, and further, SPF was measured by the following method. These results are also shown in Table 3.

(SPF Measurement Method>

These sun care products were applied to an acrylic resin (PMMA) plate, dried, and then measured at five given sites in a sample on the PMMA plate using an SPF analyzer (Labsphere UV-2000S, manufactured by Labsphere, Inc.), and the average value was calculated.
The obtained sun care product had excellent spreadability and smoothness upon application, and had a good SPF enhancing effect.

Example 21

Liquid foundation:

A liquid foundation was prepared according to the following production method using the formulation shown in the following Table 4.

A) 1 to 5 are kneaded with a roll mill.
B) 6 to 11 are uniformly mixed.
C) 12 to 15 are uniformly mixed.
D) A and B are uniformly mixed.
E) C is gradually added to D and emulsified with a homomixer (5000 rpm, 3 minutes).

[Table 4]

| | Raw material name | | Example 21 |
|---|---|---|---|
| 1 | SA-titanium CR-50 (Miyoshi Kasei) | Silicone-treated titanium oxide | 7.20 |
| 2 | SA-yellow LL-100P (Miyoshi Kasei) | Silicone-treated yellow iron oxide | 1.60 |

(continued)

| | | Raw material name | | Example 21 |
|---|---|---|---|---|
| | 3 | SA-red R-516PS (Miyoshi Kasei) | Silicone-treated red iron oxide | 0.80 |
| | 4 | SA-black BL-100P (Miyoshi Kasei) | Silicone-treated black iron oxide | 0.40 |
| | 5 | DOWSIL ES-5612 Formulation Aid (Dow Toray) | PEG-10 dimethicone | 3.00 |
| | 6 | DOWSIL 5200 Formulation Aid (Dow Toray) | Lauryl PEG/PPG-18/18 methicone | 2.00 |
| | 7 | BENTONE GEL VS-5 PC V HV (Elementis) | Disteardimonium hectorite, cyclopentasiloxane, propylene carbonate | 8.00 |
| | 8 | DOWSIL SH 245 Fluid (Dow Toray) | Cyclopentasiloxane | 29.00 |
| | 9 | FineNeo-CIO (Nippon Fine Chemical) | Cetyl ethylhexanoate | 10.00 |
| | 10 | DOWSIL EP-9215 Cosmetic Powder (Dow Toray) | (Dimethicone/vinyl dimethicone) crosspolymer | 1.00 |
| | 11 | | Composite powder of Example 1 | 2.00 |
| | 12 | | Water | 29.00 |
| | 13 | | BG | 5.00 |
| | 14 | | Sodium chloride | 0.50 |
| | 15 | PHENOXETOL (Clariant Japan) | Phenoxyethanol | 0.50 |
| | | Total | | 100.00 |

The obtained liquid foundation had excellent spreadability and smoothness upon application and had a good wrinkle blurring effect.

Example 22

Lipstick:

A lipstick was prepared according to the following production method using the formulation shown in the following Table 5.

A) 1 to 12 are melted by heating to 90°C or higher and uniformly mixed.
B) 13 to 17 are added to A and uniformly mixed and dispersed.
C) B is melted by heating to 90°C or higher, poured into a mold, and cooled to 5°C.
D) The resultant is loaded into a stick-like container.

[Table 5]

| | | | | Example 22 |
|---|---|---|---|---|
| | 1 | | Diisostearyl malate | 21.80 |
| | 2 | | Squalane | 20.00 |
| | 3 | Plandool-H (Nippon Fine Chemical) | (Phytosteryl/isostearyl/cetyl/stearyl/behenyl) dimer dilinolate | 10.00 |
| | 4 | | Isotridecyl isononanoate | 15.55 |
| | 5 | | Hydrogenated polyisobutene | 10.00 |

(continued)

|  |  |  | Example 22 |
|---|---|---|---|
| 6 | FineNeo-MCT (Nippon Fine Chemical) | Caprylic/capric triglyceride | 5.00 |
| 7 | Plandool-LG2 (Nippon Fine Chemical) | Di(phytosteryl/octyldodecyl) lauroyl glutamate | 5.00 |
| 8 |  | Polyethylene | 2.00 |
| 9 |  | Microcrystalline wax | 4.00 |
| 10 |  | Paraffin | 3.00 |
| 11 | PHENOXETOL (Clariant Japan) | Phenoxyethanol | 0.10 |
| 12 | Nipagin M (Clariant Japan) | Methylparaben | 0.10 |
| 13 |  | Polymethyl methacrylate | 1.00 |
| 14 |  | Composite powder of Example 1 | 2.00 |
| 15 |  | Red 201 | 0.20 |
| 16 |  | Red 202 | 0.20 |
| 17 |  | Yellow 4 | 0.05 |
|  | Total |  | 100.00 |

The obtained lipstick had excellent spreadability and smoothness upon application, and had a good wrinkle blurring effect.

Industrial Applicability

The composite powder of the invention has texture, light diffusing properties, and an effect such as SPF enhancement similar to those of plastic microbeads, and therefore can be used to replace plastic microbeads in a conventional cosmetic in which plastic microbeads are used.

**Claims**

1. A composite powder **characterized in that** a plate-like powder is adhered to a spherical powder coated with an oil agent.

2. The composite powder according to claim 1, wherein the spherical powder has an average particle diameter of 1 to 20 um.

3. The composite powder according to claim 1 or 2, wherein the plate-like powder has an average particle diameter of 50 um or less and an aspect ratio of 2 to 200.

4. The composite powder according to any one of claims 1 to 3, wherein the oil agent is one type or two or more types selected from semi-solid oil agents and solid oil agents at ordinary temperature.

5. The composite powder according to claim 1, wherein the oil agent is one type or two or more types selected from semi-solid oil agents and solid oil agents at ordinary temperature, the spherical powder has an average particle diameter of 1 to 20 um, and the plate-like powder has an average particle diameter of 50 um or less.

6. The composite powder according to any one of claims 1 to 5, wherein a mass ratio of the spherical powder, the plate-like powder, and the oil agent is 99.8/0.1/0.1 to 1/1/1.

7. A cosmetic **characterized by** comprising the composite powder according to any one of claims 1 to 6.

**8.** The cosmetic according to claim 7 which does not contain plastic microbeads.

**9.** A method for producing a composite powder **characterized by** comprising the following steps (a) and (b):

(a) a step of mixing and heating an oil agent, a spherical powder, and a dispersion medium, thereby obtaining the spherical powder coated with the oil agent; and
(b) a step of stirring the spherical powder coated with the oil agent and a plate-like powder, thereby obtaining a composite powder in which the plate-like powder is adhered to the spherical powder coated with the oil agent.

FIG. 1

15kV 9.8mm x4.00k BSE L          10.0μm

FIG. 2

15kV 9.7mm x3.00k BSE L          10.0μm

FIG. 3

NAME: [          ]

SAMPLE NO.[     ]

PLEASE FILL IN YOUR NAME AND SAMPLE NUMBER.
PLEASE EVALUATE SAMPLE FOR THE FOLLOWING 11 ITEMS AND FILL IN SCORE IN BOX.

| | 7 VERY | 6 MODERATELY | 5 SOMEWHAT | 4 NEITHER | 3 SOMEWHAT NOT | 2 NOT | 1 NOT AT ALL | |
|---|---|---|---|---|---|---|---|---|
| | 7 | 6 | 5 | 4 | 3 | 2 | 1 | SCORE {1 ~ 7} |

1 DRY  YES ├──┼──┼──┼──┼──┼──┤ NO   [    ]

2 COARSE  YES ├──┼──┼──┼──┼──┼──┤ NO   [    ]

3 SLIPPERY  YES ├──┼──┼──┼──┼──┼──┤ NO   [    ]

4 SILKY  YES ├──┼──┼──┼──┼──┼──┤ NO   [    ]

5 SMOOTH  YES ├──┼──┼──┼──┼──┼──┤ NO   [    ]

6 ROUGH  YES ├──┼──┼──┼──┼──┼──┤ NO   [    ]

7 SLICK  YES ├──┼──┼──┼──┼──┼──┤ NO   [    ]

8 SLIMY  YES ├──┼──┼──┼──┼──┼──┤ NO   [    ]

9 MOIST  YES ├──┼──┼──┼──┼──┼──┤ NO   [    ]

10 FLAKY  YES ├──┼──┼──┼──┼──┼──┤ NO   [    ]

11 PREFERENCE  LIKE ├──┼──┼──┼──┼──┼──┤ DISLIKE   [    ]

**INTERNATIONAL SEARCH REPORT**

| International application No. |
| --- |
| **PCT/JP2022/014232** |

**A. CLASSIFICATION OF SUBJECT MATTER**

*A61Q 1/02*(2006.01)i; *A61Q 1/06*(2006.01)i; *A61Q 1/12*(2006.01)i; *A61Q 17/04*(2006.01)i; *A61K 8/19*(2006.01)i; *A61K 8/25*(2006.01)i; *A61K 8/29*(2006.01)i; *A61K 8/37*(2006.01)i; *A61K 8/63*(2006.01)i
FI: A61K8/19; A61K8/29; A61K8/25; A61K8/63; A61K8/37; A61Q1/12; A61Q17/04; A61Q1/02; A61Q1/06

According to International Patent Classification (IPC) or to both national classification and IPC

**B. FIELDS SEARCHED**

Minimum documentation searched (classification system followed by classification symbols)

A61Q1/02; A61Q1/06; A61Q1/12; A61Q17/04; A61K8/19; A61K8/25; A61K8/29; A61K8/37; A61K8/63

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y | JP 2009-173549 A (SHISEIDO CO LTD) 06 August 2009 (2009-08-06) claims 1-2, 7-8, paragraph [0008] | 1-4, 6-9 |
| A | | 5 |
| Y | JP 8-59431 A (NOEVIR CO LTD) 05 March 1996 (1996-03-05) claim 1, paragraphs [0008], [0011], [0013]-[0015], [0030] | 1-4, 6-9 |
| A | | 5 |
| Y | JP 2010-37210 A (POLA CHEM IND INC) 18 February 2010 (2010-02-18) paragraph [0009] | 3 |

| ☐ Further documents are listed in the continuation of Box C. | ☑ See patent family annex. |
| --- | --- |

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| **25 May 2022** | **14 June 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

INTERNATIONAL SEARCH REPORT
Information on patent family members

International application No.

**PCT/JP2022/014232**

| Patent document cited in search report | | | Publication date (day/month/year) | Patent family member(s) | Publication date (day/month/year) |
|---|---|---|---|---|---|
| JP | 2009-173549 | A | 06 August 2009 | (Family: none) | |
| JP | 8-59431 | A | 05 March 1996 | (Family: none) | |
| JP | 2010-37210 | A | 18 February 2010 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020093996 A
- JP 6868468 B
- JP 6667607 B
- JP 6879276 B
- JP 6617767 B